# EUROPEAN PATENT SPECIFICATION

(11) **EP 2 769 403 B1**
(45) Date of publication and mention of the grant of the patent: **08.01.2020**
(21) Application number: 12775025.5
(22) Date of filing: 19.10.2012
(51) Int. Cl.: H01L 21/02, H01L 21/20, B82Y 15/00, B82Y 10/00, B82Y 25/00

(54) **IMPROVED BIOMARKERS AND USE THEREOF**
VERBESSERTE BIOMARKER UND IHRE VERWENDUNG
BIOMARQUEURS AMÉLIORÉS ET UTILISATION DE CES DERNIERS

(30) Priority: 19.10.2011 FR 1103184; 19.10.2011 FR 1103185; 25.05.2012 US 201261651795 P
(43) Date of publication of application: 27.08.2014
(73) Proprietor: Nexdot, 93230 Romainville (FR)
(72) Inventor: LEWINER, Jacques, 93230 Romainville (FR); MAHLER, Benoît, 93230 Romainville (FR)
(74) Representative: Icosa
(86) International application number: PCT/EP2012/070791
(87) International publication number: WO 2013/057270

(56) References cited:
- FENGSHI CAI ET AL: "TiO coated SnO nanosheet films for dye-sensitized solar cells", THIN SOLID FILMS, ELSEVIER-SEQUOIA S.A. LAUSANNE, CH, vol. 519, no. 16, 3 March 2011 (2011-03-03), pages 5645-5648, XP028089139, ISSN: 0040-6090, DOI: 10.1016/J.TSF.2011.03.013 [retrieved on 2011-03-16]
- LI Z ET AL: "Size/shape-controlled synthesis of colloidal CdSe quantum disks: Ligand and temperature effects", 4 May 2011 (2011-05-04), JOURNAL OF THE AMERICAN CHEMICAL SOCIETY 20110504 AMERICAN CHEMICAL SOCIETY USA, VOL. 133, NR. 17, PAGE(S) 6578 - 6586, XP002678847, *Sections "Introduction"; "Results"; "Discussion"; "Experimental Section"; figures 1-9*
- YI ZHANG ET AL: "Aqueous Layer-by-Layer Epitaxy of Type-II CdTe/CdSe Quantum Dots with Near-Infrared Fluorescence for Bioimaging Applications", SMALL, vol. 5, no. 2, 19 January 2009 (2009-01-19), pages 185-189, XP055050695, ISSN: 1613-6810, DOI: 10.1002/smll.200800473
- Sungjee Kim ET AL: "Type-II Quantum Dots:? CdTe/CdSe(Core/Shell) and CdSe/ZnTe(Core/Shell) Heterostructures", Journal of the American Chemical Society, vol. 125, no. 38, 29 August 2003 (2003-08-29), pages 11466-11467, XP055276844, ISSN: 0002-7863, DOI: 10.1021/ja0361749

## Description

### FIELD OF INVENTION

The present invention relates to improved fluorophores or fluorescent compounds and their use as agents in imaging or for detecting cells or analytes, in particular for biological research, medical research, diagnostic, medical imaging and medical therapy for example.

### BACKGROUND OF INVENTION

Fluorescent dyes or fluorophores have a wide variety of uses including the labeling of proteins (e.g., antibodies), DNA, carbohydrates, and cells. Fluorescent-labeled substrates have been used for visualization and/or quantitative measurements in various applications including biology, medicine, electronics, biomedicine, and forensics. Typically, conventional fluorescent dyes are used to study biochemical, pharmacological, or pathological changes that occur in tissue by labeling various cell components. Examples of conventional fluorophores which are used to detect cell components comprising lipid membranes include osmium tetraoxide, octadecyl rhodamine B, 2-hydroxyethyl7,12,17-tris (methoxyethyl) porphycene, and 1,6-diphenyl1,3,5-hexatriene. However, typically fluorophores have characteristics which interfere with their usefulness. For example, many fluorophores presently used do not have significant absorbance at the desired excitation wavelengths, or are unstable in aqueous solutions, or are unstable during illumination. More specifically, conventional fluorophores generally suffer from short-lived fluorescence; e. g., undergo photobleaching after minutes of exposure to an excitation light source. Thus, they are not very suitable for applications which require a significant length of time for ascertaining a staining pattern.

Further, conventional fluorophores are sensitive to changes in their environment which can decrease their quantum yield. Another disadvantage of conventional fluorophores is that they can only be excited in a limited range of wavelength, with a narrow spectral range providing useful absorption cross section. As a consequence, several excitation sources need to be used when several fluorophores are detected simultaneously. Another disadvantage of the conventional fluorophores is the large full width half maximum (FWHM) of their emission spectra. This disadvantage strongly limits the number of fluorophores that can be used in experiments involving multiple wavelength detections. Furthermore, conventional fluorophores are limited in sensitivity and resolution of imaging due to the limitations of intensity, photobleaching, and the finite number of molecules which can be used to label a substrate.

The brightness of a fluorophore is the optimization of three figures: the quantum yield (ratio of the number of photons emitted to the number of photons absorbed), the fluorescence lifetime (the delay between the moment a photon from the excitation source is absorbed and the moment this photon is re emitted), and the absorption cross section (the ability to absorb the excitation light). Another parameter that needs to be taken into account is the resistance to photobleaching of a fluorophore, i.e. its ability to absorb and emit several times a photon before it degrades in a non-emitting state. Another parameter that needs to be taken into account is the FWHM of the emission spectrum. With narrow FWHM a large number of fluorophores can be used simultaneously in multiple wavelengths detection experiments.

As organic fluorophores typically have low absorption cross sections, bad resistance to photobleaching, and quantum yield rather high (>50%), there is a need to provide more sensitive, versatile, reliable and brighter fluorophores useful for both single and multi-color experiments.

This has led to the development of the use of semiconductor nanocrystals, also called quantum dots, in imaging. Quantum dots are small semiconductor particles that exhibit quantum confinement. A semiconductor has a characteristic band gap, which is the difference in energy between an electron in the valence band and an electron in the conduction band of the semiconductor material. When energy is applied to the material, for example in the form of a photon having a quantum of energy greater than or equal to the band gap, an electron can be stimulated to jump from the valence band to the conduction band. The missing electron in the valence band is referred to as a "hole". When an electron falls back into a "hole" in the valence band, a photon having a quantum of energy equal to the band gap, and thus a particular wavelength, can be emitted. Thus, materials in which high energy photons can cause electrons to jump into the conduction band, after which electrons can fall back into the valence band, emitting a photon, can exhibit the phenomenon of fluorescence. Quantum confinement refers to a phenomenon observed when the physical size of the semiconductor is smaller than the typical radius of the electron-hole pair (Bohr radius). In this case, the wavelength of light emitted through electron-hole recombination is shorter than the wavelength of light emitted by the semiconductor in bulk. The wavelength of light emitted by a semiconductor exhibiting quantum confinement can be termed the characteristic wavelength. Quantum dots can be made to fluoresce at their characteristic wavelength by exposing them to light having a wavelength shorter than the characteristic wavelength. The wavelength of light emitted is dependent on the size of the quantum dot: a smaller size results in a shorter wavelength. Therefore, the characteristic wavelength of a quantum dot can be "tuned" by adjusting the size of the quantum dot. In addition, quantum dots can be produced with a narrow mono-dispersity in size, so that the light emitted from a number of quantum dots has a narrow bandwidth.

Spherical quantum dots have thus a larger absorption cross section, a better resistance to photo-bleaching, and a narrow FHWM compared to organic fluorophores. However, they present the following disadvantage: longer fluorescence lifetimes (more than 20 ns) compared to organic fluorophores.

However, despite these enhancements, quantum dots are not bright enough for several applications including Fluorescent Acquisition Cell Sorting (FACS), single molecule detection, tracking and quantification, unless their size is large (>10nm diameter). Unfortunately, nanocrystals with large diameter offer weak confinement and consequently, a restricted range of emission wavelengths can be obtained.

The present invention aims to provide new nanoparticles that are better fluorophores than the currently used, in particular for use in bioimaging.

So far, the growth of additional layer(s) on a nanosheet has not been realized: indeed the current methods for growing additional layer(s) on a nanocrystal or quantum dot were not appropriate as they led to the aggregation or destruction of the nanosheets.

The synthesis of the nanoparticles of the invention is of real interest for fluorescence or luminescence applications: indeed a bare nanosheet could not be used for these applications as their functionalization led to the loss of their fluorescence.

The inventors also found out that these nanoparticles present very interesting properties in term of stability and optical properties. Indeed, the nanoparticles of the invention present a high quantum yield, narrow FWHM (less than 30nm, preferably less than 25 nm), very fast fluorescence lifetimes (less than 2 ns to less than 10 ns), large absorption cross section (more than 10⁻¹³ cm² at 400 nm), tunable emission wavelength and good resistance to photobleaching; and are therefore very useful as fluorescent agent.

Thanks to their large absorption cross section and their very fast fluorescence lifetimes, the nanoparticles of the invention thus present a greater luminescent intensity or brightness than conventional nanocrystals. In addition, their narrow FWHM is a great advantage in multiplex detection experiments as a large number of nanoparticles can be used simultaneously.

### SUMMARY

One object of the invention is a nanoparticle according to claim 1.

In one embodiment, the nanoparticle has a thickness from 0.5 nm to 50 nm.

In one embodiment, the nanoparticle has lateral dimensions at least 1.5 times larger than its thickness.

In one embodiment, the nanoparticle comprises at least one semi-conductor from group IV, group III-V, group II-VI, group I-III-VI, group II-V, group III-VI.

In one embodiment, the nanosheet and the at least one layer comprise a material MxEy, wherein
M is Zn, Cd, Hg, Cu, Ag, Al, Ga, In, Si, Ge, Pb, Sb or a mixture thereof,
E is O, S, Se, Te, N, P, As or a mixture thereof, and
x and y are independently a decimal number from 0 to 5, at the condition that x and y are not 0 at the same time.

According to the invention, the nanoparticle has a structure core/shell where the nanosheet and the at least one layer do not have the same composition.

Another object of the invention is the use a nanoparticle as described here above as a fluorophore, a fluorescent agent, a marker or a probe.

Another object of the invention is a fluorophore conjugate comprising at least one nanoparticle as described here above associated to a specific-binding component.

In one embodiment, said specific-binding component is selected among antigens, steroids, vitamins, drugs, haptens, metabolites, toxins, environmental pollutants, amino acids, peptides, proteins, antibodies, polysaccharides, nucleotides, nucleosides, nucleic acids, nucleic acid polymers, carbohydrates, lipids, phospholipids, polymers, lipophilic polymers, polymeric microparticle, cells and virus.

Another object of the invention is the use of the nanoparticle as described here above or the fluorophore conjugate as described here above in a detection system.

Another object of the invention is a method for detecting an analyte in a sample, said method comprising:
(a) contacting said sample with a fluorophore conjugate as described here above, wherein the component is a binding partner for said analyte;
(b) incubating said conjugate with said sample for a sufficient amount of time for said analyte and said component to interact, thereby forming a fluorescent analyte; and
(c) illuminating said fluorescent analyte with an appropriate wavelength, whereby the presence of said analyte is determined in said sample.

Another object of the invention is a method for detecting multiple analytes in a sample, said method comprising:
(a) contacting said sample with multiples fluorophore conjugate as described here above, wherein each component is a binding partner for one analyte and wherein each fluorophore presents a different fluorescent emission;
(b) incubating said conjugates with said sample for a sufficient amount of time for said analytes and said components to interact, thereby forming multiple fluorescent analytes; and
(c) illuminating said fluorescent analytes with an appropriate wavelength, whereby the presence of said analyte is determined in said sample.

Another object of the invention is a kit for detection of at least one analyte in a sample comprising at least one nanoparticle as described here above or at least one fluorophore conjugate as described here above.

Another object of the invention is the use of the nanoparticle as described here above or the fluorophore conjugate as described here above, in fluorescence detection methods or as biosensors.

Another object of the invention is the use of the nanoparticle as described here above or the fluorophore conjugate as described here above, for in vivo animal imaging or for ex vivo live cells imaging.

### DEFINITIONS

In the present invention, the following terms have the following meanings:
The terms "a" or "an" means "at least one" or "one or more".

The terms "nanosheet" or "nanoplatelets" or "NPL" or "nanodisk" have the same meaning in the present invention.

The terms "polynucleotide", "oligonucleotide", "nucleic acid" and "nucleic acid molecule" are used herein to include a polymeric form of nucleotides of any length, either ribonucleotides or deoxyribonucleotides. This term refers only to the primary structure of the molecule. Thus, the term includes triple-, double- and single stranded DNA, as well as triple-, double- and single-stranded RNA. It also includes modifications, such as by methylation and/or by capping, and unmodified forms of the polynucleotide. More particularly, the terms "polynucleotide", "oligonucleotide", "nucleic acid" and "nucleic acid molecule" include polydeoxyribonucleotides (containing 2-deoxy-D-ribose), polyribonucleotides (containing D-ribose), any other type of polynucleotide which is an Nor C-glycoside of a purine or pyrimidine base, and other polymers containing nonnucleotidic backbones, for example, polyamide (e. g., peptide nucleic acids (PNAs)) and polymorpholino (polymers, and other synthetic sequence-specific nucleic acid polymers providing that the polymers contain nucleobases in a configuration which allows for base pairing and base stacking, such as is found in DNA and RNA). There is no intended distinction in length between the terms "polynucleotide", "oligonucleotide", "nucleic acid" and "nucleic acid molecule", and these terms will be used interchangeably. These terms refer only to the primary structure of the molecule. Thus, these terms include, for example, 3'deoxy-2', 5'-DNA, oligodeoxyribonucleotide N3'P5'phosphoramidates, 2'-O-alkyl- substituted RNA, double-and single-stranded DNA, as well as double- and single-stranded RNA, DNA: RNA hybrids, and hybrids between PNAs and DNA or RNA, and also include known types of modifications, for example, labels which are known in the art, methylation, "caps", substitution of one or more of the naturally occurring nucleotides with an analog, internucleotide modifications such as, for example, those with uncharged linkages (e. g., methyl phosphonates, phosphotriesters, phosphoramidates, carbamates, etc.), with negatively charged linkages (e. g., phosphorothioates, phosphorodithioates, etc.), and with positively charged linkages (e. g., aminoalklyphosphoramidates, aminoalkylphosphotriesters), those containing pendant moieties, such as, for example, proteins (including nucleases, toxins, antibodies, signal peptides, poly-L-lysine, etc.), those with intercalators (e. g., acridine, psoralen, etc.), those containing chelators (e. g., metals, radioactive metals, boron, oxidative metals, etc.), those containing alkylators, those with modified linkages (e. g., alpha anomeric nucleic acids, etc.), as well as unmodified forms of the polynucleotide or oligonucleotide. In particular, DNA is deoxyribonucleic acid.

The term "aptamer" (or nucleic acid antibody) is used herein to refer to a single-or doublestranded DNA or a single-stranded RNA molecule that recognizes and binds to a desired target molecule by virtue of its shape.

"Polypeptide" and "protein" are used interchangeably herein and include a molecular chain of amino acids linked through peptide bonds. The terms do not refer to a specific length of the product. Thus, "peptides", "oligopeptides" and "proteins" are included within the definition of polypeptide. The terms include post-translational modifications of the polypeptide, for example, glycosylations, acetylations, phosphorylations and the like. In addition, protein fragments, analogs, mutated or variant proteins, fusion proteins and the like are included within the meaning of polypeptide.

The terms "specific-binding molecule" and "affinity molecule" are used interchangeably herein and refer to a molecule that will selectively bind, through chemical or physical means to a detectable substance present in a sample. By "selectively bind" is meant that the molecule binds preferentially to the target of interest or binds with greater affinity to the target than to other molecules. For example, an antibody will selectively bind to the antigen against which it was raised; a DNA molecule will bind to a substantially complementary sequence and not to unrelated sequences. The affinity molecule can comprise any molecule, or portion of any molecule, that is capable of being linked to a fluorophore of the invention and that, when so linked, is capable of recognizing specifically a detectable substance. Such affinity molecules include, by way of example, such classes of substances as antibodies, as defined below, monomeric or polymeric nucleic acids, aptamers, proteins, polysaccharides, sugars, and the like.

The term "antibody" is used herein includes antibodies obtained from both polyclonal and monoclonal preparations, as well as, the following: hybrid (chimeric) antibody molecules; F (ab') 2 and F (ab) fragments; Fv molecules; single-chain Fv molecules (sFv); dimeric and trimeric antibody fragment constructs; minibodies; and, any functional fragments obtained from such molecules, wherein such fragments retain specific binding properties of the parent antibody molecule.

The nanoparticle fluorophore of the invention is "linked" or "conjugated" to, or "associated" with, a specific-binding molecule or member of a binding pair when the fluorophore is chemically coupled to, or associated with the specific binding molecule. Thus, these terms intend that the fluorophore of the invention may either be directly linked to the specific-binding molecule or may be linked via a linker moiety, such as via a chemical linker described below. The terms indicate items that are physically linked by, for example, covalent chemical bonds, physical forces such van der Waals or hydrophobic interactions, encapsulation, embedding, or the like. As an example without limiting the scope of the invention, the fluorophore of the invention can be conjugated to molecules that can interact physically with biological compounds such as cells, proteins, nucleic acids, subcellular organelles and other subcellular components. For example, the fluorophore of the invention can be associated with biotin which can bind to the proteins, avidin and streptavidin. Also, the fluorophore of the invention can be associated with molecules that bind nonspecifically or sequence-specifically to nucleic acids (DNA, RNA). As examples without limiting the scope of the invention, such molecules include small molecules that bind to the minor groove of DNA, small molecules that form adducts with DNA and RNA; cisplatin, molecules that intercalate between the base pairs of DNA (e. g. methidium, propidium, ethidium, porphyrins, etc., radiomimetic DNA damaging agents such as bleomycin, neocarzinostatin and other enediynes and metal complexes that bind and/or damage nucleic acids through oxidation; chemical and photochemical probes of DNA.

As used herein, a "biological sample" refers to a sample of isolated cells, tissue or fluid, including but not limited to, for example, plasma, serum, spinal fluid, semen, lymph fluid, the external sections of the skin, respiratory, intestinal, and genitourinary tracts, tears, saliva, milk, blood cells, tumors, organs, and also samples of in vitro cell culture constituents (including but not limited to conditioned medium resulting from the growth of cells in cell culture medium, putatively virally infected cells, recombinant cells, and cell components).

A "biomolecule" is a synthetic or naturally occurring molecule, such as a protein, amino acid, nucleic acid, nucleotide, carbohydrate, sugar, lipid and the like.

A "homogeneous assay" is one that is performed without transfer, separation or washing steps. Thus, for example, a homogeneous High Throughput Screening (HTS) assay involves the addition of reagents to a vessel, e. g., a test tube or sample well, followed by the detection of the results from that particular well. A homogeneous HTS assay can be performed in the solution in the test tube or well, on the surface of the test tube or well, on beads or cells which are placed into the test tube or the well, or the like. The detection system typically used is a fluorescence, chemiluminescence, or scintillation detection system.

The term "multiplexing" is used herein to include conducting an assay or other analytical method in which multiple analytes or biological states can be detected simultaneously by using more than one detectable label, each of which emits at a distinct wavelength, with a distinct intensity, with a distinct FWHM, with a distinct fluorescence lifetime, or any combination thereof. Preferably, each detectable label is linked to one of a plurality of first members of binding pairs each of which first members is capable of binding to a distinct corresponding second member of the binding pair. A multiplexed method using the fluorophores of the invention having distinct emission spectra can be used to detect simultaneously in the range of 2 to 1,000,000, preferably in the range of 2 to 10,000, more preferably in the range of 2 to 100, or any integer between these ranges, and even more preferably in the range of up to 10 to 20, e. g., 2, 3, 4, 5, 6, 7, 8, 9, 10, 11, 12, 13, 14, 15, 16, 17, 18, 19, or 20, of analytes, biological compounds or biological states. Multiplexing also includes assays or methods in which the combination of more than one fluorophores of the invention having distinct emission spectra can be used to detect a single analyte. The term "barcode" as used herein refers to one or more sizes, size distributions, compositions, or any combination thereof, of the fluorophores of the invention. Each size, size distribution and/or composition of the fluorophores of the invention has a characteristic emission spectrum, e. g., wavelength, intensity, FWHM, and/or fluorescent lifetime. In addition to the ability to tune the emission energy by controlling the size of the particular fluorophore of the invention, the intensities of that particular emission observed at a specific wavelength are also capable of being varied, thus increasing the potential information density provided by the fluorophore barcode system. In preferred embodiments, 2-15 different intensities may be achieved for a particular emission at a desired wavelength, however, one of ordinary skill in the art will realize that more than fifteen different intensities may be achieved, depending upon the particular application of interest. For the purposes of the present invention, different intensities may be achieved by varying the concentrations of the particular size of the fluorophore of the invention attached to, embedded within or associated with an item, compound or matter of interest. The "barcode" enables the determination of the location or identity of a particular item, compound or matter of interest. For example, the fluorophores of the invention can be used to barcode chromosomes, as well as portions of chromosomes, for spectral karyotyping, as described further below.

### DETAILED DESCRIPTION

Due to their properties as mentioned here above, the nanoparticles of the invention are particularly useful as fluorophores, fluorescent agents, probes or markers, in particular for biological and medical research and imaging.
Due to their properties as mentioned here above, the nanoparticles of the invention are particularly useful as agents in bio-imaging.

The present invention thus relates to a nanoparticle comprising a nanosheet coated partially or totally by at least one layer of inorganic material.

In one embodiment, at least one face of the nanosheet is coated by at least one layer of inorganic material.
According to the invention, the term "layer" refers to a continuous or partial layer or film having a thickness of at least one atom. The term "monolayer" corresponds to a continuous or partial layer or film having a thickness of one atom. The atoms present in the layer or monolayer can be identical or different.
In one embodiment, the nanosheet is a colloidal inorganic nanosheet.
In one embodiment, the nanosheet is crystalline.
In one embodiment, the nanoparticle of the invention is a nanosheet.
In one embodiment, the nanoparticle of the invention is inorganic.
In one embodiment, the nanoparticle of the invention is colloidal.
In one embodiment, the nanoparticle of the invention is crystalline.
In one embodiment, the nanosheet comprised in the nanoparticle of the invention still has its quasi-2D structure. According to the invention, the nanoparticle of the invention has a core/shell structure, i.e. the nanosheet is partially or totally coated by at least one layer of inorganic material.
In another embodiment, the nanoparticle of the invention comprises a nanosheet totally coated by a first layer of inorganic material, said first layer being partially or totally coated by at least one further layer of inorganic material.

In one embodiment, the nanoparticle of the invention has an absorption cross section that can be tuned to arbitrarily large values while their emission wavelength remains unchanged.
In one embodiment, the nanosheet comprised in the nanoparticle of the invention has a thickness of about 0.3 nm to about 50 nm, about 0.3 nm to about 40 nm, about 0.3 nm to about 30 nm, about 0.3 nm to about 20 nm, about 0.3 nm to about 10 nm, about 0.3 nm to about 5 nm, about 0.3 nm to about 2 nm.
In one embodiment, the nanosheet comprised in the nanoparticle of the invention has lateral dimensions (length and/or width) of at least 1.5 times its thickness.
In another embodiment, the lateral dimensions of the nanosheet are at least 2, 2.5, 3, 3.5, 4, 4.5, 5 times larger than its thickness.

In another embodiment of the invention, the lateral dimensions of the nanosheet are at least 2, 2.5, 3, 3.5, 4.5, 5, 6, 7, 8, 9, 10 times larger than the thickness.
In another embodiment of the invention, the lateral dimensions are of the nanosheet at least 20, 30, 40, 50, 60, 70, 80, 90, 100, 150, 200, 250, 300, 350, 400, 450, 500 times larger than the thickness.
In one embodiment, the lateral dimensions of the nanosheet are from at least 0.45 nm to at least 500 nm.
In one embodiment, the lateral dimensions of the nanosheet are from at least 2 nm to less than 50 nm, from 2 nm to 25 nm, from 2 nm to 20 nm, from 2 nm to 15 nm, from 2 nm to 10 nm, from 2 nm to 8 nm, from 2 nm to 6 nm.
In one embodiment, the nanosheet comprised in the nanoparticle of the invention is a semi-conductor from group IV, group III-V, group II-VI, group I-III-VI, group II-V, group III-VI.
In one embodiment, the nanosheet comprised in the nanoparticle of the invention comprises a material MxEy, wherein
M is Zn, Cd, Hg, Cu, Ag, Al, Ga, In, Si, Ge, Pb, Sb or a mixture thereof,
E is O, S, Se, Te, N, P, As or a mixture thereof, and
x and y are independently a decimal number from 0 to 5, at the condition that when x is 0, y is not 0 and inversely.
In one embodiment, the nanosheet comprised in the nanoparticle of the invention comprises a material from Si, Ge, CdS, CdSe, CdTe, ZnS, ZnSe, ZnTe, HgS, HgSe, HgTe, PbS, PbSe, PbTe, CuInS₂, CuInSe₂, AgInS₂, AgInSe₂, CuS, Cu₂S, Ag₂S, Ag₂Se, Ag₂Te, InN, InP, InAs, InSb, In₂S₃, Cd₃P₂, Zn₃P₂, Cd₃As₂, Zn₃As₂, ZnO, AlN, AlP, AlAs, AlSb, GaN, GaP, GaAs, GaSb, and alloys and mixtures thereof.

In one embodiment, the nanoparticle of the invention has a thickness of about 0.5 nm to about 50 nm, about 0.5 nm to about 40 nm, about 0.5 nm to about 30 nm, about 0.5 nm to about 20 nm, about 0.5 nm to about 10 nm, about 0.5 nm to about 5 nm, about 0.5 nm to about 2 nm.
In one embodiment, the nanoparticle of the invention has lateral dimensions (length and/or width) of at least 1.5 times its thickness.

In another embodiment, the lateral dimensions of the nanoparticle of the invention are at least 2, 2.5, 3, 3.5, 4, 4.5, 5 times larger than its thickness.
In another embodiment of the invention, the lateral dimensions of the nanoparticle of the invention are at least 2, 2.5, 3, 3.5, 4.5, 5, 6, 7, 8, 9, 10 times larger than the thickness. In another embodiment of the invention, the lateral dimensions of the nanoparticle of the invention are at least 20, 30, 40, 50, 60, 70, 80, 90, 100, 150, 200, 250, 300, 350, 400, 450, 500 times larger than the thickness.
In one embodiment, the lateral dimensions of the nanoparticle of the invention are from at least 0.75 nm to at least 500 nm.
In one embodiment, the lateral dimensions of the nanoparticle of the invention are from at least 2 nm to 50 nm, from 2 nm to 25 nm, from 2 nm to 20 nm, from 2 nm to 15 nm, from 2 nm to 10 nm, from 2 nm to 8 nm, from 2 nm to 6 nm.
According to the invention, the nanoparticle of the invention comprises a semi-conductor selected from group IV, group III-V, group II-VI, group I-III-VI, group II-V, group III-VI.
In one embodiment, the nanoparticle of the invention comprises a material MxEy, wherein
M is Zn, Cd, Hg, Cu, Ag, Al, Ga, In, Si, Ge, Pb, Sb or a mixture thereof,
E is O, S, Se, Te, N, P, As or a mixture thereof, and
x and y are independently a decimal number from 0 to 5, at the condition that when x is 0, y is not 0 and inversely.

In one embodiment, the nanoparticle of the invention comprises a material from Si, Ge, CdS, CdSe, CdTe, ZnS, ZnSe, ZnTe, HgS, HgSe, HgTe, PbS, PbSe, PbTe, CuInS₂, CuInSe₂, AgInS₂, AgInSe₂, CuS, Cu₂S, Ag₂S, Ag₂Se, Ag₂Te, InN, InP, InAs, InSb, In₂S₃, Cd₃P₂, Zn₃P₂, Cd₃As₂, Zn₃As₂, ZnO, AlN, AlP, AlAs, AlSb, GaN, GaP, GaAs, GaSb, and alloys and mixtures thereof.

In one embodiment, the nanoparticle of the invention comprises a core which is coated partially or totally with at least one layer of inorganic material.
According to the invention, the nanoparticle of the invention is a core/shell type structure, i.e. having a core surrounded by a shell made of at least one layer of inorganic material.

In one embodiment, the core and the at least one layer of inorganic material are composed of the same material or are composed of different material.
In one embodiment, the core and the at least one layer of inorganic material may be a semi-conductor from group IV, group III-V, group II-VI, group I-III-VI, group II-V, group III-VI.
In another embodiment, the core and the at least one layer of inorganic material may comprise a material MxEy, wherein
M is Zn, Cd, Hg, Cu, Ag, Al, Ga, In, Si, Ge, Pb, Sb or a mixture thereof,
E is O, S, Se, Te, N, P, As or a mixture thereof, and
x and y are independently a decimal number from 0 to 5, at the condition that when x is 0, y is not 0 and inversely.
In another embodiment, the core and the at least one layer of inorganic material may be composed of a material from Si, Ge, CdS, CdSe, CdTe, ZnS, ZnSe, ZnTe, HgS, HgSe, HgTe, PbS, PbSe, PbTe, CuInS₂, CuInSe₂, AgInS₂, AgInSe₂, CuS, Cu₂S, Ag₂S, Ag₂Se, Ag₂Te, InN, InP, InAs, InSb, In₂S₃, Cd₃P₂, Zn₃P₂, Cd₃As₂, Zn₃As₂, ZnO, AlN, AlP, AlAs, AlSb, GaN, GaP, GaAs, GaSb, and alloys and mixtures thereof.
In another embodiment of the invention, the nanoparticle of the invention may be further surrounded by a "coat" of an organic capping agent. The organic capping agent may be any number of materials, but has an affinity for the semiconductor surface. In general, the capping agent can be an isolated organic molecule, a polymer (or a monomer for a polymerization reaction), an inorganic complex, and an extended crystalline structure.

In one embodiment of the invention, examples of the nanoparticle of the invention include, but are not limited to, nanosheet of CdSe with a shell of CdS or ZnS (having a quantum yield superior to 60% and a narrow FHWM (less than 20 nm)), nanosheet of CdS coated with ZnS (CdS/Zns), nanosheet of CdSeₓS₁₋ₓ (x being a decimal number from 0 to 1) coated with Cd_{y}Zn_{1-y}S (y being a decimal number from 0 to 1) (CdSeS/CdZnS), nanosheet of CdSeₓSe₁₋ₓ (x being a decimal number from 0 to 1) coated with ZnS (CdSeS/ZnS), nanosheet of CdTe coated with Cd_{y}Zn_{1-y}SeₓS₁₋ₓ ((x and y being independently a decimal number from 0 to 1) CdTE/CdZnSeS), nanosheet of CdS coated with ZnS doped with Manganese II or Copper II CdS/ZnS:Mn or CdS/ZnS:Cu.

Preferably, the nanoparticle of the invention for use as a fluorophore, fluorescent agent or marker is selected in the group comprising CdSe, CdTe, CdS, and the core/shell structures such as CdSe/CdS, CdSe/CdZnS, CdTe/CdS/CdZnS, CdS/ZnS .

According to the invention, the nanoparticle of the invention can be obtained by two methods: a one-pot method and a layer-by-layer method.

According to the invention, the one-pot method for obtaining the nanoparticle of the invention comprises the step of contacting at least one nanosheet with at least one precursor of the material to be deposited on the nanosheet.
In one embodiment, the precursor of the material to be deposited is a precursor of a material MxEy, wherein
M is Zn, Cd, Hg, Cu, Ag, Al, Ga, In, Si, Ge, Pb, Sb or a mixture thereof,
E is O, S, Se, Te, N, P, As or a mixture thereof, and
x and y are independently a decimal number from 0 to 5, at the condition that when x is 0, y is not 0 and inversely.
In one embodiment, the material MxEy comprises cationic elements M and anionic elements E in stoichiometric ratio, said stoichiometric ratio being characterized by values of x and y corresponding to absolute values of mean oxidation number of elements E and M respectively.

According to the invention, the layer-by-layer method for obtaining the nanoparticle of the invention comprises the step of
(a) contacting at least one nanosheet with a precursor of the element R to be deposited, said precursor having a positive oxidation number when the nanosheet has a negative surface charge and a negative oxidation number when the nanosheet has a positive surface charge,
(b) waiting a time t for the reaction of R precursor with the surface atoms of the nanosheet,
(c) optionally, eliminate after reaction the surplus of precursor.

In one embodiment, the precursor of the element R to be deposited is a precursor of either M or E as defined here above, wherein when the nanosheet or the layer to be coated is M, then the precursor is a precursor of E and inversely.
In one embodiment, the precursor of element R is introduced in sufficient quantity to be in stoichiometric proportions with the surface atoms of the nanosheet or of the layer to be coated (in order to obtain a continuous coating).
In one embodiment, the precursor of element R is introduced in sub-stoichiometric quantities (in order to obtain a partial coating).
In one embodiment, the 2 or 3 steps of the layer-by-layer method are repeated n times, n being an integer superior or equal to 1, thus allowing the coating of the nanosheet with multiple monolayers MₙEₙ₊₁Mₙ₊₂Eₙ₊₃...or EₙMₙ₊₁Eₙ₊₂Mₙ₊₃..., M and E being identical or different in composition.

In one embodiment, the at least one precursor of the material or of the element R to be deposited is in solution.
In one embodiment, the nanosheet is in a medium, preferably an organic medium, preferably apolar or weakly polar.
In one embodiment, the nanosheet is dispersed in a solvent.
In one embodiment, said solvent is chosen among distilled water, methanol, ethanol, isopropanol, butanol, chloroform, acetone, hexane, tetrahydrofurane, dimethylsulfoxyde, toluene, octadecene, dimethylformamide.
In one embodiment, said solvent is a supercritical fluid or a ionic fluid.

In one embodiment, if E is a chalcogen, the precursor of E is a compound containing the chalcogen with an oxidation number -2.
In one embodiment, if E is sulfur, the precursor of E is a salt containing S²⁻ ions.
In one embodiment, the precursor of E comprises Bis(trimethylsilyl) sulfide (TMS₂S), hydrogen sulfide (H₂S), sodium hydrosulfide (NaSH) or sodium sulfide (Na₂S), ammonium sulfide (S(NH₄)₂), thiourea or thioacetamide.
In one embodiment, if E is selenium, the precursor of E is a salt containing Se²⁻ ions.

In one embodiment, the precursor of E comprises Bis(trimethylsilyl) selenide (TMS₂Se), hydrogen selenide (H₂Se), sodium hydroselenide (NaSeH) or sodium selenite (Na₂SeO₃), or selenourea.
In one embodiment, if E is tellurium, the precursor of E is a salt containing Te²⁻ ions.
In one embodiment, the precursor of E comprises Bis(trimethylsilyl) telluride (TMS₂Te), hydrogen telluride (H₂Te), sodium hydrotelluride (NaTeH) or sodium tellurite (Na₂TeO₃). In one embodiment, if E is a chalcogen, the precursor of E is formed in situ by reaction of a reducing agent with a compound containing E with an oxidation number of 0 or a strictly positive oxidation number.
In one embodiment, if E is oxygen, the precursor of E is HO⁻.
In one embodiment, if E is oxygen, the precursor of E is a sodium hydroxide solution (NaOH) or a potassium hydroxide solution (KOH) or a tetramethylammonium hydroxide solution (TMAOH).
In one embodiment, if E is phosphorus, the precursor of E comprises phosphorus with an oxidation number of -3.
In one embodiment, the precursor of E comprises tris(trimethylsilyl) phosphine (TMS₃P), or phosphine (PH₃) or white phosphorus (P₄) or phosphorus trichloride (PCl₃).
In one embodiment, if M is a metal, the precursor of M is a compound containing the metal with a positive or null number degree.
In one embodiment, if M is a metal, the precurseur of M comprises a metallic salt.
In one embodiment, the metallic salt is M carboxylate, M chloride, M nitrate, M sulfate or M thiolate.
In one embodiment, the film or layer of MxEy composition deposited comprises a chalcogen, a phosphide, a nitride, an arsenide or an oxide.
In one embodiment, the film or layer deposited comprises an alloy of the above mentioned materials.
In one embodiment, the film or layer deposited comprises a transition metal or a lanthanide in minor quantity (doping).
In one embodiment, the film or layer deposited comprises in minor quantities an element producing an excess or a deficit of electrons compared to the film or layer alone (electronic doping).
In one embodiment, the film or layer deposited comprises a metal.

In one embodiment, the metal deposited is chosen among gold, silver, nickel, molybdenum and aluminium.
In one embodiment, a reducing agent can be introduced at the same time than the precursor(s) of the film or layer to be deposited.
In one embodiment, the reducing agent comprises an hydride.
In one embodiment, the reducing agent comprises dihydrogen.
In one embodiment, the hydride comprises sodium borohydride (NaBH₄), sodium hydride (NaH), lithium aluminium hydride (LiAlH₄), diisobutylaluminium hydride (DIbAlH).

In one embodiment, a stabilization compound capable of stabilizing the nanoparticle of the invention in solution can be introduced in the medium or dispersion solvent.
In one embodiment, the stabilization compound is an organic ligand.
In one embodiment, the organic ligand comprises a carboxylic acid, a thiol, an amine, a phosphine, an amide, an ester, a pyridine, an imidazole or an alcool.
In one embodiment, the organic ligand is an ion.
In one embodiment, the stabilization ion is a quaternary ammonium.

In one embodiment, the introduction flow rate of the precursor solution(s) is chosen such as the thickness growth of the nanosheet is comprised from 1 nm/s to 0.1 nm/h.
In one embodiment, the introduction flow rate of the precursor solution(s) is chosen such as the thickness growth of the nanosheet is comprised from 1 nm/s to 0.1 nm/day.
In one embodiment, the precursor solution(s) is added dropwise.
In one embodiment, the precursor solution(s) is added in one pot in the reaction medium. In one embodiment, the temperature of the reaction medium is from -100°C to +350°C.
In one embodiment, the temperature of the reaction medium is from -80°C to +150°C, from +20°C to +100 °C, from +40°C to +80°C, about +60°C (±5°C).
In one embodiment, the temperature of the reaction medium is room temperature.

In one embodiment, the reaction time t of the precursor is less than few hours.
In one embodiment, the reaction time t of the precursor is less than 24h, less than 12h, less than 6h, less than 4h, less than 2h, less than 1h.
In one embodiment, the reaction time t of the precursor is less than few days.

In one embodiment, the surplus of precursor is eliminated after reaction, optionally by washing.
In one embodiment, the nanoparticle of the invention is purified after reaction.
In one embodiment, the purification is carried out by flocculation or filtration.

In one embodiment, the nanosheet can be fixed on at least one substrate.
In one embodiment, said fixation may be carried out by adsorption or chemical coupling.
In one embodiment, said substrate may be chosen among silicon dioxide SiO₂, aluminium oxide Al₂O₃, indium tin oxide ITO, fluorine-doped tin oxide (FTO), titanium dioxide TiO₂, gold, silver, nickel, molybdenum, aluminium, silicon, germanium, silicon carbide SiC, graphene and cellulose.
In one embodiment, said substrate comprises a polymer.
In one embodiment, the method can further comprise a step of annealing to smooth and homogenate the surface of the nanoparticle of the invention.

In one embodiment, the surface of the nanoparticle of the invention can be modified to produce a fluorophore that can be coupled to a variety of biological molecules or substrates by techniques well known in the art.

The present invention provides chemical and biological assays which use the nanoparticle fluorophore of the invention as detectable luminescent labels to detect the presence or amount of one or more molecules, biomolecules or analyte, as well as to detect biological interactions, biological processes, alterations in biological processes, or alterations in the structure of a chemical or biological compound.
The nanoparticle fluorophore of the invention can be used to detect or track a single target. Additionally, a population of fluorophores of the invention may be used for either simultaneous detection of multiple targets or to detect particular compounds and/or items of interest in, e. g., a library of compounds. For example, compositions of nanoparticles fluorophores of the invention comprising one or more particle size distributions having characteristic spectral emissions may be used as "barcodes" in assays to either track the location or source of a particular item of interest or to identify a particular item of interest.

The fluorophores of the invention used in such a "barcoding" scheme can be tuned to a desired wavelength to produce a characteristic spectral emission by changing their composition and size, or size distribution. Additionally, the intensity of the emission at a particular characteristic wavelength can also be varied, thus enabling the use of binary or higher order encoding schemes.
The information encoded by the nanoparticles fluorophores of the invention can be spectroscopically decoded, thus providing the location and/or identity of the particular item or component of interest.
The nanoparticles fluorophores of the invention can be used to detect the presence and/or amount of a biological moiety, e. g., a biological target analyte; the structure, composition, and conformation of a biological molecule; the localization of a biological moiety, e. g., a biological target analyte in an environment; interactions of biological molecules; alterations in structures of biological compounds; and/or alterations in biological processes.
Thus, it is readily apparent that the nanoparticles fluorophores of the invention find use in a variety of assays where other, less reliable, labeling methods have typically been used, including, without limitation, fluorescence microscopy, histology, cytology, pathology, flow cytometry, western blotting, Fluorescence Resonance Energy Transfer (FRET), immunocytochemistry, Fluorescence In Situ Hybridization (FISH) and other nucleic acid hybridization assays, signal amplification assays, DNA and protein sequencing, immunoassays such as competitive binding assays and ELISAs, immunohistochemical analysis, protein and nucleic acid separation, homogeneous assays, multiplexing, high throughput screening, chromosome karyotyping, and the like.

Another object of the invention is a composition comprising the nanoparticle fluorophore of the invention associated with a specific-binding component, such that the composition can detect the presence and/or amounts of biological and chemical compounds, detect interactions in biological systems, detect biological processes, detect alterations in biological processes, or detect alterations in the structure of biological compounds. Without limitation, the fluorophore conjugates comprise any component linked to the nanoparticle fluorophore of the invention that can interact with a biological target, to detect biological processes, or reactions, as well as alter biological molecules or processes.

Another object of the invention is a fluorophore conjugate resulting from attachment of the nanoparticle fluorophore of the invention and a component as defined here after.
In one embodiment, said component may be chosen among antigens, steroids, vitamins, drugs, haptens, metabolites, toxins, environmental pollutants, amino acids, peptides, proteins, nucleic acids, nucleic acid polymers, carbohydrates, lipids, and polymers. In another embodiment, said component may be chosen among an amino acid, peptide, protein, antibody, polysaccharide, nucleotide, nucleoside, oligonucleotide, nucleic acid, hapten, a psoralen, drug, a hormone, lipid, phospholipid, lipoprotein, lipopolysaccharide, liposome, lipophilic polymer, a synthetic polymer, polymeric microparticle, biological cell, a virus and combinations thereof.
In one embodiment of the invention, said component is labeled with a plurality of fluorophores of the invention, which may be the same or different.
In one embodiment, said component comprises an amino acid (including those that are protected or are substituted by phosphates, carbohydrates, or C1 to C22 carboxylic acids), or a polymer of amino acids such as a peptide or protein. Preferred conjugates of peptides contain at least five amino acids, more preferably 5 to 36 amino acids. Preferred peptides include, but are not limited to, neuropeptides, cytokines, toxins, protease substrates, and protein kinase substrates. Also preferred are peptides that serve as organelle localization peptides, that is, peptides that serve to target the conjugated compound for localization within a particular cellular substructure by cellular transport mechanisms. Preferred protein conjugates include enzymes, antibodies, lectins, glycoproteins, histones, albumins, lipoproteins, avidin, streptavidin, protein A, protein G, phycobiliproteins and other fluorescent proteins, hormones, toxins and growth factors. Typically, the conjugated protein is an antibody, an antibody fragment, avidin, streptavidin, a toxin, a lectin, or a growth factor.
In another embodiment, said component comprises a nucleic acid base, nucleoside, nucleotide or a nucleic acid polymer. Preferred nucleic acid polymer conjugates are single-or multi-stranded, natural or synthetic DNA or RNA oligonucleotides, or DNA/RNA hybrids, or incorporating an unusual linker such as morpholine derivatized phosphates, or peptide nucleic acids such as N-(2-aminoethyl)glycine units, where the nucleic acid contains fewer than 50 nucleotides, more typically fewer than 25 nucleotides.

In another embodiment, said component comprises a carbohydrate or polyol that is typically a polysaccharide, such as dextran, FICOLL, heparin, glycogen, amylopectin, mannan, inulin, starch, agarose and cellulose, or is a polymer such as a poly(ethylene glycol).
In another embodiment, said component comprises a lipid (typically having 6-25 carbons), including glycolipids, phospholipids, and sphingolipids. Alternatively, said molecule or molecular complex comprises a lipid vesicle, such as a liposome, or is a lipoprotein. Some lipophilic substituents are useful for facilitating transport of the fluorophore of the invention into cells or cellular organelles.
In another embodiment, said component includes polymers, polymeric particles, polymeric microparticles including magnetic and non-magnetic microspheres, polymeric membranes, conducting and non-conducting metals and non-metals, and glass and plastic surfaces and particles. Conjugates are typically prepared by chemical modification of a polymer that contains functional groups with suitable chemical reactivity. The conjugated polymer may be organic or inorganic, natural or synthetic. In a preferred embodiment, the present compounds are conjugated to a polymer matrix, such as a polymeric particle or membrane, including membranes suitable for blot assays for nucleic acids or proteins. In another embodiment, said molecule or molecular complex comprises a glass or silica, which may be formed into an optical fiber or other structure. In another embodiment, said molecule or molecular complex comprises a poly(ethylene glycol), a poly(acrylate) or a poly(acrylamide).
Alternatively, the conjugates of the present invention are conjugates of cells, cellular systems, cellular fragments, or subcellular particles. Examples of this type of conjugated material include virus particles, bacterial particles, virus components, biological cells (such as animal cells, plant cells, bacteria, or yeast), or cellular components. Examples of cellular components that can be labeled, or whose constituent molecules can be labeled, include but are not limited to lysosomes, endosomes, cytoplasm, nuclei, histones, mitochondria, Golgi apparatus, endoplasmic reticulum and vacuoles.

The fluorophore conjugates can be made using techniques known in the art. For example, moieties such as Tri octyl phosphine oxide (TOPO) and tri octyl phosphine (TOP), may be readily displaced and replaced with other functional moieties, including, but not limited to carboxylic acids, amines, aldehydes, and styrene to name a few. The person skilled in the art will realize that factors relevant to the success of a particular displacement reaction include the concentration of the replacement moiety, temperature and reactivity.
The ability to utilize a general displacement reaction to modify selectively the surface functionality of the fluorophore of the invention enables functionalization for specific uses. For example, because detection of biological compounds is most preferably carried out in aqueous media, a preferred embodiment of the present invention utilizes nanoparticles fluorophores of the invention that are solubilized in water. In the case of water-soluble fluorophores, the outer layer includes a compound having at least one linking moiety that attaches to the surface of the nanoparticle and that terminates in at least one hydrophilic moiety. The linking and hydrophilic moieties are spanned by a hydrophobic region sufficient to prevent charge transfer across the region. The hydrophobic region also provides a "pseudohydrophobic" environment for the fluorophore and thereby shields it from aqueous surroundings. The hydrophilic moiety may be a polar or charged (positive or negative) group. The polarity or charge of the group provides the necessary hydrophilic interactions with water to provide stable solutions or suspensions of the nanoparticles of the invention. Exemplary hydrophilic groups include polar groups such as hydroxides (-OH), amines, polyethers, such as polyethylene glycol and the like, as well as charged groups, such as carboxylates (-CO2⁻), sulfonates (-SO3⁻), phosphonates (-PO4²⁻), nitrates, ammonium salts (NH4⁺), and the like. A water-solubilizing layer is found at the outer surface of the overcoating layer. Methods for rendering the fluorophore of the invention water-soluble are known in the art.
Additional modifications can also be made such that the nanoparticle fluorophore of the invention can be associated with almost any solid support. A solid support, for the purposes of this invention, is defined as an insoluble material to which compounds are attached during a synthesis sequence, screening, immunoassays, etc. The use of a solid support is particularly advantageous for the synthesis of libraries because the isolation of support-bound reaction products can be accomplished simply by washing away reagents from the support-bound material and therefore the reaction can be driven to completion by the use of excess reagents. A solid support can be any material that is an insoluble matrix and can have a rigid or semi-rigid surface. Exemplary solid supports include but are not limited to pellets, disks, capillaries, hollow fibers, needles, pins, solid fibers, cellulose beads, pore-glass beads, silica gels, polystyrene beads optionally cross-linked with divinylbenzene, grafted co-poly beads, polyacrylamide beads, latex beads, dimethylacrylamide beads optionally crosslinked with N-N'-bis acryloylethylenediamine, and glass particles coated with a hydrophobic polymer.
For example, the fluorophore of the invention can readily be functionalized to create styrene or acrylate moieties, thus enabling its incorporation into polystyrene, polyacrylate or other polymers such as polyimide, polyacrylamide, polyethylene, polyvinyl, polydiacetylene, polyphenylene-vinylene, polypeptide, polysaccharide, polysulfone, polypyrrole, polyimidazole, polythiophene, polyether, epoxies, silica glass, silica gel, siloxane, polyphosphate, hydrogel, agarose, cellulose, and the like.
Examples of fluorophore conjugate include, but are not limited to, fluorophore streptavidine conjugate, mouse IgG2a fluorophore conjugate, fluorophore anti-fluorescein conjugate, CD2 mAb (monoclonal antibody) fluorophore conjugate, CD3 mAb fluorophore conjugate, CD4 mAb fluorophore conjugate, CD8 mAb fluorophore conjugate, CD14 mAb fluorophore conjugate, CD19 mAb fluorophore conjugate, CD20 mAb fluorophore conjugate, CD25 mAb fluorophore conjugate, CD27 mAb fluorophore conjugate, CD45 mAb fluorophore conjugate, CD45R mAb fluorophore conjugate, CD45RA mAb fluorophore conjugate, CD56 mAb fluorophore conjugate, HLA DR mAb fluorophore conjugate, fluorophore donkey anti-goat IgG conjugate, fluorophore donkey anti-mouse IgG conjugate, fluorophore donkey anti-rabbit IgG conjugate, fluorophore goat F(ab')2 anti-mouse IgG, fluorophore goat F(ab')2 anti-rabbit IgG, fluorophore wheat germ agglutinin (WGA) conjugate.
Examples of nanoparticle fluorophore functionalized ready to be used for conjugation include, but are not limited to, carboxylate functionalized fluorophore and amino (PEG) fluorophore.

Another object of the invention is the use of a nanoparticle fluorophore or a fluorophore conjugate of the invention in a detection system, wherein said detection system includes, but is not limited to, an affinity assay, fluorescent staining, flow cytometry, nucleic acid sequencing, nucleic acid hybridization, nucleic acid synthesis or amplification, or molecular sorting.

Another object of the invention is a method for detecting an analyte in a sample, preferably a biological sample, said method comprising:
(a) contacting said sample with a conjugate as defined here above, wherein the component is a binding partner for said analyte;
(b) incubating said conjugate with said sample for a sufficient amount of time for said analyte and said component to interact, thereby forming a fluorescent analyte; and
(c) illuminating said fluorescent analyte with an appropriate wavelength, whereby the presence of said analyte is determined in said sample.

At any time after or during an assay or staining procedure, the sample is illuminated with a wavelength of light that results in a detectable optical response, and observed with a means for detecting the optical response. The nanoparticles fluorophores of the invention are detected upon illumination, such as by an ultraviolet or visible wavelength emission lamp, an arc lamp, or a laser. Selected equipment that is useful for illuminating the fluorophore conjugates of the invention includes, but is not limited to, hand-held ultraviolet lamps, mercury arc lamps, xenon lamps, argon lasers, laser diodes, and YAG lasers. These illumination sources are optionally integrated into laser scanners, fluorescence microplate readers, standard or mini fluorometers, or chromatographic detectors. This fluorescence emission is optionally detected by visual inspection, or by use of any of the following devices: CCD cameras, video cameras, photographic film, laser scanning devices, fluorometers, photodiodes, quantum counters, epifluorescence microscopes, scanning microscopes, flow cytometers, fluorescence microplate readers, or by means for amplifying the signal such as photomultiplier tubes. Where the sample is examined using an instrument such as a flow cytometer, a fluorescence microscope or a fluorometer, the instrument is optionally used to distinguish and discriminate between distincts fluorophores with detectably different optical properties, typically by distinguishing the fluorescence response of one fluorophore conjugate from another one. Where the sample is examined using a flow cytometer, examination of the sample optionally includes isolation of particles within the sample based on the fluorescence response of the fluorophore by using a sorting device.
A detectable optical response means a change in, or occurrence of, a parameter in a test system that is capable of being perceived, either by direct observation or instrumentally. Such detectable responses include the change in, or appearance of, color, fluorescence, reflectance, chemiluminescence, light polarization, light scattering, or x-ray scattering. Typically the detectable response is a change in fluorescence, such as a change in the intensity, excitation or emission wavelength distribution of fluorescence, fluorescence lifetime, fluorescence polarization, or a combination thereof. The detectable optical response may occur throughout the sample or in a localized portion of the sample. The presence or absence of the optical response after the elapsed time is indicative of one or more characteristic of the sample. Comparison of the degree of staining with a standard or expected response can be used to determine whether and to what degree the sample possesses a given characteristic.

In another embodiment, the nanoparticles fluorophores or the fluorophore conjugates of the invention may be used in a multiplex assay for detecting one or more species in a mixture. As used herein, the term "multiplex assay" refers to an assay in which fluorescence from two or more fluorophores is detected, or in which fluorescence energy transfer between two or more fluorophores and one or more quencher is detected.
Another object of the invention is a method for detecting multiple analytes in a sample, preferably a biological sample, said method comprising:
(a) contacting said sample with multiple conjugates as defined here above, wherein each component is a binding partner for one analyte and wherein each fluorophore presents a different fluorescent emission;
(b) incubating said conjugates with said sample for a sufficient amount of time for said analytes and said components to interact, thereby forming multiple fluorescent analytes; and
(c) illuminating said fluorescent analytes with an appropriate wavelength, whereby the presence of said analytes is determined in said sample.

Another object of the invention is a kit for detection of at least one analyte in a sample, wherein said kit comprises at least one nanoparticle fluorophore as defined here above or at least one fluorophore conjugate as defined here above.

The nanoparticles fluorophores of the invention can be used in the following fluorescence detection methods: FACS, multicolor optical coding of cells, microarrays, immunochemistry, multiplex FISH, fixed cell or tissue imaging.
The nanoparticles fluorophores of the invention can also be used as biosensors: tagged antibodies, FRET sensors, encoded multiplexed microbeads.
Another use for the nanoparticles fluorophores of the invention is in vivo animal imaging (cells, tissues, organs, tumors) for example in the context of photo-induced therapy, optical surgical aid or pharmacokinetic determination of therapeutic agents.
The fluorophores of the invention can also be used for ex vivo live cells imaging.

### Brief description of the figures

**Figure 1****.** Absorption and emission spectra of a CdSe nanosheets solution in hexane.
**Figure 2****.** Transmission Electron Microscopy (TEM) image of CdSe nanosheets.
**Figure 3****.** Absorption and emission spectra of a CdSe/CdZnS nanoparticles solution in Chloroform.
**Figure 4****.** Transmission Electron Microscopy (TEM) image of CdSe/CdZnS nanoparticles.
**Figure 5****.** Fluorescence lifetime of CdSe/CdZnS nanoparticles.
**Figure 6****.** Fluorescence lifetime comparison between CdSe/CdZnS nanoparticles and CdSe/CdZnS core/shell quantum dots.
**Figure 7****.** Absorption and emission spectra of encapsulated CdSe/CdZnS nanoparticles in micelles.
**Figure 8****.** Transmission Electron Microscopy (TEM) image of encapsulated CdSe/CdZnS nanoparticles in micelles.
**Figure 9****.** Fluorescence lifetime of encapsulated CdSe/CdZnS nanoparticles in micelles.

### Examples

### CdSe nanosheets synthesis.

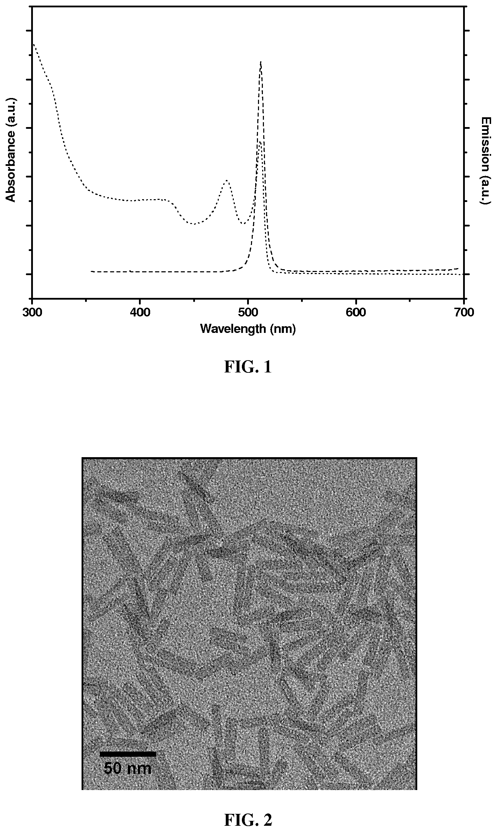

90 mL of 1-octadecene, 480 mg of Cadmium acetate dihydrate (Cd(OAc)₂(H₂O)₂) and 1.18 g of oleic acid were introduced into a 250 mL three-neck flask. The mixture was degassed under vacuum and magnetic stirring at 110 °C during 90 minutes. After inerting the flask with Argon, 72 mg of elemental Se powder dispersed in 2 mL ODE was swiftly injected in the hot mixture and the temperature was set to 240 °C. When the temperature reached 205 °C, 240 mg of Cd(OAc)₂(H₂0)₂ was introduced in the reaction mixture and the reaction was allowed to proceed 15 minutes at 240 °C. The mixture was then cooled down to room temperature and 10 mL oleic acid was added, inducing the aggregation of the platelets. The mixture was centrifuged 10 minutes at 5000 rpm, the supernatant was discarded and the precipitated nanosheets were suspended in hexane. The nanosheets were then precipitated one more time with ethanol and suspended in 10 mL hexane. The nanosheets are characterized by absorption and emission spectroscopy (Figure 1) and by Transmission Electron Microscopy (Figure 2).

### CdS shell growth.

400 µL of the as-synthetized nanosheets in hexane were diluted in 2 mL chloroform. 20 mg of thioacetamide (TAA) and 200 µL of octylamine were added in the flask and the mixture was sonicated until the complete dissolution of the TAA (about 5 minutes). The color of the solution changed from yellow to orange in about 10 minutes. 200 µL of a 0.1 M Cd(oleate)₂ solution in octylamine was then added to the reaction mixture. The reaction was allowed to proceed for 3 hours at room temperature. An important secondary nucleation occurred but the growth of the CdS shell on the initial CdSe nanosheets was still effective. After synthesis, the nanoparticles were isolated from the secondary nucleation by precipitation with a few drops or ethanol and suspended in CHCl₃. To improve the quantum yield and the dispersion, the final core/shell platelets were capped with Cd(oleate)₂ and insolated with UV light for one hour.

### Cd_{0.7}Zn_{0.3}S shell growth.

1 mL of the as-synthetized nanosheets in hexane were diluted in 4 mL chloroform. 100 mg of thioacetamide (TAA) and 1 mL of octylamine were added in the flask and the mixture was sonicated until the complete dissolution of the TAA (about 5 minutes). The color of the solution changed from yellow to orange during this time. 350 µL of a solution of Cd(NO₃)₂ 0.2 M in ethanol and 150 µL of a solution of Zn(NO₃)₂ 0.2 M in ethanol are then added to the flask. The reaction was allowed to proceed for 24 hours at room temperature. After synthesis, the nanoparticles were isolated from the secondary nucleation by precipitation with a few drops or ethanol and suspended in 5 mL CHCl₃. To improve the stabilization and the quantum yield of the nanoparticles, 100 µL of Zn(NO₃)₂ 0.2 M in ethanol is added to the NPLs solution. They aggregate steadily and are resuspended by adding 200 µL oleic acid. At this time their quantum yield is low and is recovered over few days.

### Cd_{0.33}Zn_{0.66}S shell growth.

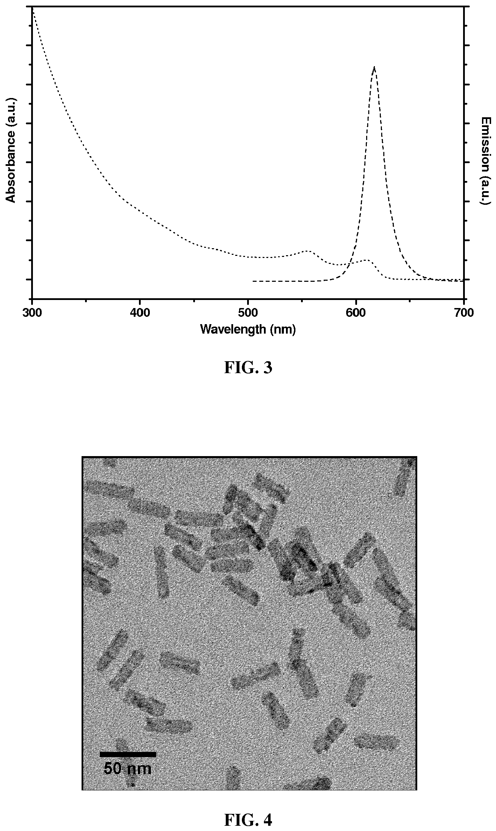

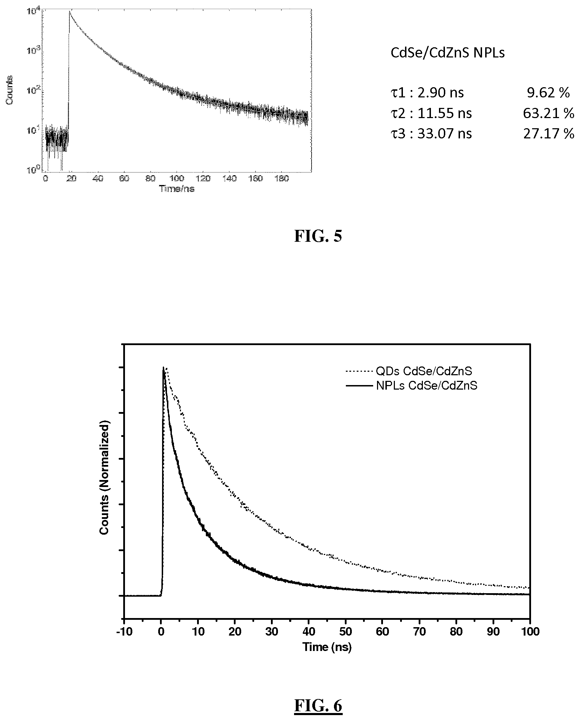

500 µL of the as-synthetized nanosheets in hexane were diluted in 4 mL chloroform. 30 mg of thioacetamide (TAA) and 500 µL of octylamine were added in the flask and the mixture was sonicated until the complete dissolution of the TAA (about 5 minutes). The color of the solution changed from yellow to orange during this time. 50 µL of a solution of Cd(NO₃)₂ 0.2 M in ethanol and 100 µL of a solution of Zn(NO₃)₂ 0.2 M in ethanol are then added to the flask. The reaction was allowed to proceed for 3 hours at 60°C. After synthesis, the nanoparticles were isolated from the secondary nucleation by precipitation with a few drops or ethanol and suspended in 5 mL CHCl₃. To improve the stabilization and the quantum yield of the nanoparticles, 20 µL of Cd(oleate)₂ in octylamine (100mg/mL) is added to the nanoparticles solution. At this time their quantum yield is low and is recovered over few days. The nanoparticles are characterized by absorption and emission spectroscopy (Figure 3) and by Transmission Electron Microscopy (Figure 4). Their fluorescence lifetime is measured (Figure 5) and is compared to CdSe/CdZnS core/shell quantum dots (Figure 6).

### Nanoparticles encapsulation in phospholipid micelles.

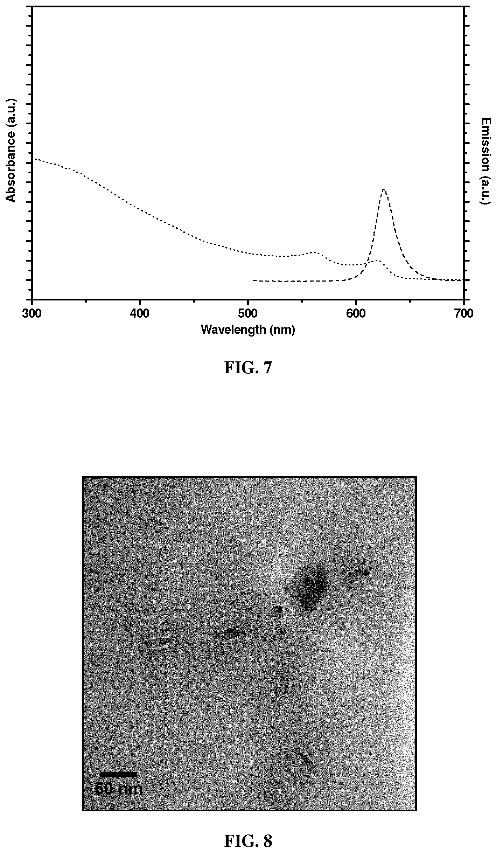

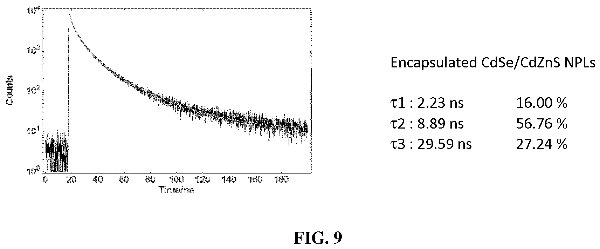

In a Wheaton flask, 30 µl of 10 µM nanoparticles solution in chloroform and 200 µl of the desired PEG2000- phospholipid solution (20 mg/ml) (e.g., 200 µl mPEG(2000) PE lipid solution for nonfunctionalized micelles or 180 µl mPEG(2000) PE and 20 µl DSPE-PEG(2000)-NH2 lipid solutions for further bioconjugation) were mixed together. The resulting solution was sonicated for 5 minutes before adding 1 mL of MilliQ water. Then the biphasic mixer was heated at 80°C by a regularized temperature bath to remove the chloroform. After the complete evaporation of the chloroform the nanoparticles were encapsulated and well dispersed in water. The resulting solution was transferred in a microtube and the possible aggregated nanoparticles were eliminated by a rapid centrifugation at 10,000g at 20°C - 25°C for 1 min. To separate the nanoparticles solution from the residue of aggregates, the supernatant was collected into a Sartorius Vivaspin (Mw cutoff 10 kDa), then the volume of the nanoparticles solution was reduced to 200 µl by centrifugation at 10,000g at 20 °C - 25°C for 5 min. The encapsulated nanoparticles are characterized by absorption and emission spectroscopy (Figure 7) and by Transmission Electron Microscopy (Figure 8). Their fluorescence lifetime is measured (Figure 9).

### Conjugation of encapsulated nanoparticles to DNA.

Conjugation of encapsulated nanoparticles with DNA was obtained from the encapsulated nanoparticles made with previous procedure using 180 µl mPEG(2000) PE and 20 µl DSPE-PEG(2000)-NH2 lipid solutions. The DNA contained a disulfide group at the 5' end. The disulfide bond was cleaved with dithiothreitol (DTT) and the oligonucleotide was purified of excess DTT. The coupling of the DNA to the nanoparticles-micelle was performed using Sulfosuccinimidyl 4-(Nmaleimidomethyl)cyclohexane-1-carboxylate (SulfoSMCC).

### Ligand exchange with DHLA-SB (Dihydrolipoic Acid - Sulfobetaine).

Nanoparticles in hexane (8 nmol) were precipitated with ethanol. The supernatant was removed and the nanoparticles were redispersed in 3mL of chloroform. This solution was vigorously stirred with a fresh aqueous solution of DHLA-SB (∼100 mg) at room temperature. Phase transfer of nanoparticles from chloroform to water occurs very quickly in less than 30 minutes which is indicative of effective cap exchange. After about 1 hour, the vial containing the biphasic mixture was sealed and stored without stirring at 60°C overnight to complete cap exchange. The nanoparticles aqueous solution was extracted from the mixture and heated to evaporate residual organic solvent. Excess free solubilized ligands and reagents were removed by two rounds of membrane ultrafiltration at 10,000g using a Sartorius Vivaspin disposable filter (cutoff 10 kDa) in 20 mM NaCl. Then, nanoparticles-SB were purified by ultracentrifugation at 30,000g for 30 minutes in a 10 - 40% sucrose gradient in 20 mM NaCl. The NCs band was collected and residual sucrose was removed by two rounds of ultrafiltration (Sartorius Vivaspin, cutoff 10 kDa) in 20 mM NaCl.

### Conjugation of nanoparticles-SB to Streptavidin or Biotin.

Nanoparticles with a mixed sulfobetaine / COOH surface were obtained as indicated above with a 1:1 DHLA-SB: DHLA-PEG600-COOH ratio. For the conjugation to streptavidin (SA), these nanoparticles were first activated with 10000 equivalents of 1-Ethyl-3-(3-dimethylaminopropyl) carbodiimide hydrochloride (EDC) / N-hydroxysuccinimide (NHS) in 2-(N-morpholino)ethanesulfonic acid (MES) buffer (100 mM, pH 6.2) for 30 min. Excess coupling reagent was removed twice by ultrafiltration through a 10 kDa cutoff filter (Vivaspin). Activated nanoparticles were then mixed with SA (50 equivalents) in sodium bicarbonate buffer (200 mM, pH 8.1) and incubated for 1 h. The nanoparticles-SA were concentrated by ultrafiltration through a 10 kDa cutoff filter and then purified by ultracentifugation on a 10 - 40% sucrose gradient with 20mM NaCl. The gradient was performed with a gradient maker (Biocomp) and the sample was ultracentrifuged at 30,000g during 30 minutes. Then the band was collected with the pump of the gradient maker. The efficiency of the conjugation was checked mixing during 15 minutes 6 pmol of NCs-SA with biotin agarose beads. The beads were washed four times in PBS by centrifugation. The control was performed mixing nanoparticles-SA with streptavidin agarose beads.

Nanoparticles-biotin were obtained by cap exchange with 1:1 DHLA-SB:DHLA-PEG600-biotin. Nanoparticles-biotin were purified by ultracentrifugation as indicated above for nanoparticles-SA and the functionality of biotin was tested on streptavidin agarose beads with a control on biotin agarose beads.

## Claims

1. A nanoparticle comprising a nanosheet coated partially or totally with at least one layer of inorganic material, wherein the nanoparticle comprises at least one semiconductor selected from group IV, group III-V, group II-VI, group I-III-VI, group II-V, group III-VI; and wherein the nanoparticle has a core/shell structure wherein the nanosheet and the at least one layer do not have the same composition, **characterized in that** said nanosheet has a lateral dimension which is at least 3 times larger than its thickness.

2. The nanoparticle according to claim **1**, having a thickness from 0.5 nm to 50 nm.

3. The nanoparticle according to claim **1** or **2**, wherein its lateral dimensions is at least 1.5 times larger than its thickness.

4. The nanoparticle according to any one of claim **1** to **3**, wherein the nanosheet and the at least one layer comprise a material MₓE_{y}, wherein M is Zn, Cd, Hg, Cu, Ag, Al, Ga, In, Si, Ge, Pb, Sb or a mixture thereof, E is O, S, Se, Te, N, P, As or a mixture thereof, and x and y are independently a decimal number from 0 to 5, at the condition that x and y are not 0 at the same time.

5. Use of a nanoparticle according to any one of claim **1** to **4**, as a fluorophore, a fluorescent agent, a marker or a probe.

6. A fluorophore conjugate comprising at least one nanoparticle according to any one of claims **1** to **4** associated to a specific-binding component.

7. The fluorophore conjugate according to claim **6**, wherein said specific-binding component is selected among antigens, steroids, vitamins, drugs, haptens, metabolites, toxins, environmental pollutants, amino acids, peptides, proteins, antibodies, polysaccharides, nucleotides, nucleosides, nucleic acids, nucleic acid polymers, carbohydrates, lipids, phospholipids, polymers, lipophilic polymers, polymeric microparticle, cells and virus.

8. Use of the nanoparticle according to any one of claims **1** to **4** or the fluorophore conjugate according to any one of claims **6** to **7** in a detection system.

9. A method for detecting an analyte in a sample, said method comprising:
a) contacting said sample with a fluorophore conjugate according to any one of claims **6** to **7**, wherein the component is a binding partner for said analyte;
b) incubating said conjugate with said sample for a sufficient amount of time for said analyte and said component to interact, thereby forming a fluorescent analyte; and
c) illuminating said fluorescent analyte with an appropriate wavelength, whereby the presence of said analyte is determined in said sample.

10. A method for detecting multiple analytes in a sample, said method comprising:
a) contacting said sample with multiple fluorophore conjugates according to any one of claims **6** to **7**, wherein each component is a binding partner for one analyte and wherein each fluorophore presents a different fluorescent emission;
b) incubating said conjugates with said sample for a sufficient amount of time for said analytes and said components to interact, thereby forming multiple fluorescent analytes; and
c) illuminating said fluorescent analytes with an appropriate wavelength, whereby the presence of said analyte is determined in said sample.

11. A kit for detection of at least one analyte in a sample, the kit comprising at least one nanoparticle according to any one of claims **1** to **4** or at least one fluorophore conjugate according to any one of claims **6** to **7.**

12. Use of the nanoparticle according to any one of claims **1** to **4** or the fluorophore conjugate according to any one of claims **6** to **7**, in fluorescence detection methods or as biosensors.

13. Use of the nanoparticle according to any one of claims **1** to **4** or the fluorophore conjugate according to any one of claims **6** to **7**, for in vivo animal imaging or for ex vivo live cells imaging.

## Patentansprüche

1. Nanoteilchen, das ein Nanosheet umfasst, wobei das Nanosheet teilweise oder vollständig mit mindestens einer Schicht aus anorganischem Material überzogen ist, wobei das Nanoteilchen mindestens einen Halbleiter umfasst, der aus Gruppe IV, Gruppe III-V, Gruppe II-VI, Gruppe I-III-VI, Gruppe II-V, Gruppe III-VI ausgewählt ist; und wobei das Nanoteilchen eine Kern-/Schale-Struktur aufweist, wobei das Nanosheet und die mindestens eine Schicht nicht dieselbe Zusammensetzung aufweisen, **dadurch gekennzeichnet, dass** das Nanosheet eine Seitenabmessung aufweist, die mindestens 3-mal größer ist als ihre Dicke.

2. Nanoteilchen nach Anspruch **1,** das eine Dicke von 0,5 nm bis 50 nm aufweist.

3. Nanoteilchen nach Anspruch **1** oder **2,** wobei seine Seitenabmessungen mindestens 1,5-mal größer ist als seine Dicke.

4. Nanoteilchen nach einem aus Anspruch **1** bis **3**, wobei das Nanosheet und die mindestens eine Schicht ein MₓE_{y}-Material umfassen, wobei M Zn, Cd, Hg, Cu, Ag, Al, Ga, In, Si, Ge, Pb, Sb oder eine Mischung davon ist, E O, S, Se, Te, N, P, As oder eine Mischung davon ist, und x und y unabhängig eine Dezimalzahl von 0 bis 5 sind, mit der Bedingung, dass x und y nicht gleichzeitig 0 sind.

5. Verwendung eines Nanoteilchens nach einem aus Anspruch **1** bis **4** als ein Fluorophor, ein Fluoreszenzmittel, einen Marker oder eine Sonde.

6. Fluorophor-Konjugat, das mindestens ein Nanoteilchen nach einem der Ansprüche **1** bis **4** umfasst, das mit einer spezifisch bindenden Komponente assoziiert ist.

7. Fluorophor-Konjugat nach Anspruch **6**, wobei die spezifisch bindende Komponente ausgewählt ist aus Antigenen, Steroiden, Vitaminen, Arzneimitteln, Haptenen, Metaboliten, Toxinen, Umweltschadstoffen, Aminosäuren, Peptiden, Proteinen, Antikörpern, Polysacchariden, Nukleotiden, Nukleosiden, Nukleinsäuren, Nukleinsäurepolymeren, Kohlenhydraten, Lipiden, Phospholipiden, Polymeren, lipophilen Polymeren, polymeren Mikroteilchen, Zellen und Viren.

8. Verwendung des Nanoteilchens nach einem der Ansprüche **1** bis **4** oder des Fluorophor-Konjugats nach einem der Ansprüche **6** bis **7** in einem Nachweissystem.

9. Verfahren zum Nachweisen eines Analyten in einer Probe, wobei das Verfahren umfasst:
a) Inkontaktbringen der Probe mit einem Fluorophor-Konjugat nach einem der Ansprüche **6** bis **7**, wobei die Komponente ein Bindungspartner für den Analyten ist;
b) Inkubieren des Konjugats mit der Probe über eine ausreichende Zeitdauer, damit der Analyt und die Komponente interagieren, wodurch ein fluoreszierender Analyt gebildet wird; und
c) Beleuchten des fluoreszierenden Analyten mit einer geeigneten Wellenlänge, wodurch das Vorhandensein des Analyten in der Probe bestimmt wird.

10. Verfahren zum Nachweisen mehrerer Analyten in einer Probe, wobei das Verfahren umfasst:
a) Inkontaktbringen der Probe mit mehreren Fluorophor-Konjugaten nach einem der Ansprüche **6** bis **7**, wobei jede Komponente ein Bindungspartner für einen Analyten ist, und wobei jedes Fluorophor eine unterschiedliche Fluoreszenzemission aufweist;
b) Inkubieren der Konjugate mit der Probe über eine ausreichende Zeitdauer, damit die Analyten und die Komponenten interagieren, wodurch mehrere fluoreszierende Analyten gebildet werden; und
c) Beleuchten der fluoreszierenden Analyten mit einer geeigneten Wellenlänge, wodurch das Vorhandensein des Analyten in der Probe bestimmt wird.

11. Set zum Nachweisen mindestens eines Analyten in einer Probe, wobei das Set mindestens ein Nanoteilchen nach einem der Ansprüche **1** bis **4** oder mindestens ein Fluorophor-Konjugat nach einem der Ansprüche **6** bis **7** umfasst.

12. Verwendung des Nanoteilchens nach einem der Ansprüche **1** bis **4** oder des Fluorophor-Konjugats nach einem der Ansprüche **6** bis **7** in Fluoreszenznachweisverfahren oder als Biosensoren.

13. Verwendung des Nanoteilchens nach einem der Ansprüche **1** bis **4** oder des Fluorophor-Konjugats nach einem der Ansprüche **6** bis **7** zur In-vivo-Bildgebung an Tieren oder zur Ex-vivo-Bildgebung an lebenden Zellen.

## Revendications

1. Une nanoparticule comprenant un nanofeuillet, ledit nanofeuillet étant couvert partiellement ou totalement avec au moins une couche de matériau inorganique, dans laquelle la nanoparticule comprend au moins un semiconducteur choisi parmi le groupe IV, le groupe III-V, le groupe II-VI, le groupe I-III-VI, le groupe II-V, le groupe III-VI; et dans laquelle la nanoparticule a une structure cœur/coque dans laquelle le nanofeuillet et la au moins une couche n'ont pas la même composition, **caractérisée en ce que** ledit nanofeuillet a une dimension latérale qui est au moins 3 fois plus grande que son épaisseur.

2. La nanoparticule selon la revendication **1**, ayant une épaisseur allant de 0,5 nm à 50 nm.

3. La nanoparticule selon la revendication **1** ou **2**, dans laquelle ses dimensions latérales sont au moins 1,5 fois plus grandes que son épaisseur.

4. La nanoparticule selon l'une quelconque des revendications **1** à **3**, dans laquelle le nanofeuillet et la au moins une couche comprennent un matériau MₓE_{y}, dans lequel M est Zn, Cd, Hg, Cu, Ag, Al, Ga, In, Si, Ge, Pb, Sb ou un mélange de ceux-ci, E est O, S, Se, Te, N, P, As ou un mélange de ceux-ci, et x et y sont indépendamment un nombre décimal entre 0 et 5, à la condition que x et y ne soient pas 0 en même temps.

5. Utilisation d'une nanoparticule selon l'une quelconque des revendications **1** à **4**, comme fluorophore, agent fluorescent, marqueur ou sonde.

6. Un fluorophore conjugué comprenant au moins une nanoparticule selon l'une quelconque des revendications **1** à **4** associée à un composant à liaison spécifique.

7. Le fluorophore conjugué selon la revendication **6**, dans lequel ledit composant à liaison spécifique est choisi parmi des antigènes, des stéroïdes, des vitamines, des médicaments, des haptènes, des métabolites, des toxines, des polluants environnementaux, des acides aminés, des peptides, des protéines, des anticorps, des polysaccharides, des nucléotides, des nucléosides, des acides nucléiques, des polymères d'acide nucléique, des glucides, des lipides, des phospholipides, des polymères, des polymères lipophiles, des microparticules polymériques, des cellules et des virus.

8. Utilisation de la nanoparticule selon l'une quelconque des revendications **1** à **4** ou du fluorophore conjugué selon l'une quelconque des revendications **6** à **7** dans un système de détection.

9. Une méthode pour détecter un analyte dans un échantillon, ladite méthode comprenant :
a) mettre en contact l'échantillon et un fluorophore conjugué selon l'une quelconque des revendications **6** à **7**, dans lequel le composant est un partenaire de liaison pour ledit analyte ;
b) incuber ledit conjugué avec ledit échantillon pendant une période de temps suffisante pour que ledit analyte et ledit composant interagissent, formant ainsi un analyte fluorescent ; et
c) illuminer ledit analyte fluorescent avec une longueur d'onde appropriée, par laquelle la présence dudit analyte dans ledit échantillon est déterminée.

10. Une méthode pour détecter plusieurs analytes dans un échantillon, ladite méthode comprenant :
a) mettre en contact l'échantillon et plusieurs fluorophores conjugués selon l'une quelconque des revendications **6** à **7**, dans lequel chaque composant est un partenaire de liaison pour un analyte et dans lequel chaque fluorophore présente une émission fluorescente différente ;
b) incuber lesdits conjugués avec ledit échantillon pendant une période de temps suffisante pour que lesdits analytes et lesdits composants interagissent, formant ainsi plusieurs analytes fluorescents ; et
c) illuminer lesdits analytes fluorescents avec une longueur d'onde appropriée, par laquelle la présence dudit analyte dans ledit échantillon est déterminée.

11. Un kit pour la détection d'au moins un analyte dans un échantillon, le kit comprenant au moins une nanoparticule selon l'une quelconque des revendications **1** à **4** ou au moins un fluorophore conjugué selon l'une quelconque des revendications **6** à **7.**

12. Utilisation de la nanoparticule selon l'une quelconque des revendications **1** à **4** ou du fluorophore conjugué selon l'une quelconque des revendications **6** à **7** dans des méthodes de détection par fluorescence ou comme biocapteurs.

13. Utilisation de la nanoparticule selon l'une quelconque des revendications **1** à **4** ou du fluorophore conjugué selon l'une quelconque des revendications **6** à **7**, pour de l'imagerie animale in vivo ou pour de l'imagerie ex vivo de cellules vivantes.
